# EUROPÄISCHE PATENTSCHRIFT

(11) **EP 2 055 276 B1**
(45) Veröffentlichungstag und Bekanntmachung des Hinweises auf die Patenterteilung: **15.07.2015**
(21) Anmeldenummer: 08019222.2
(22) Anmeldetag: 03.11.2008
(51) Int. Cl.: A61F 11/08

(54) **Gehörschutz**
Hearing protection
Protection auditive

(30) Priorität: 02.11.2007 DE 102007052780; 12.03.2008 DE 102008013848
(43) Veröffentlichungstag der Anmeldung: 06.05.2009
(73) Patentinhaber: Janßen, Dirk, 67122 Altrip (DE)
(72) Erfinder: Janßen, Dirk, 67122 Altrip (DE); Potak, Sandor, 63743 Aschaffenburg (DE)
(74) Vertreter: Dosterschill, Peter

(56) Entgegenhaltungen:
- WO-A1-99/36016
- FR-A1- 2 558 055
- FR-A1- 2 842 726
- US-A- 5 819 745
- US-A1- 2004 129 276

## Beschreibung

Die Erfindung bezieht sich auf einen Gehörschutz zum Druckausgleich für Flugreisende gemäß Oberbegriff des Anspruchs 1, wie er von Flugreisenden zum Druckausgleich beim Steig- und Sinkflug verwendet wird.

In Verkehrsflugzeugen herrscht nach Erreichen der Reiseflughöhe ein Kabineninnendruck von 700 bis 800 h/Pa. Dies entspricht dem Luftdruckniveau auf einem Berg mit einer Höhe von rund 2000 bis 3000 Metern ü.N.N. Der Luftdruck am Boden auf Meereshöhe beträgt je nach Witterungslage rund 1000 h/Pa.

Für das menschliche Ohr kann ein sich rasch verändernder Luftdruck eine sehr hohe Belastung sein. Das Trommelfell ist nämlich luftdicht und schützt so die Wahrnehmungsorgane im Mittelohr. Eine Belüftung des Mittelohres geschieht dabei durch die Eustachische Röhre; sie muss auch Druckunterschiede zwischen Mittelohr und Umwelt ausgleichen.

Während des Steigfluges (Startphase) sinkt der Kabineninnendruck relativ rasch ab, d. h. innerhalb von 15 bis 30 Minuten auf 700 bis 800 h/Pa. Dieser Druckabfall ist in der Regel für das Ohr problemlos, da die Eustachische Röhre einen relativen Überdruck im Mittelohr nur passiv ablassen muss. Während des Sinkfluges steigt der Kabineninnendruck ebenfalls rasch an, d. h. in 15 bis 30 Minuten auf Bodendruck, wobei die Anstiegsrate des Kabineninnendruckes abhängig ist vom Baumuster des Flugzeuges, vom vorgeschriebenen Landeweg sowie vom Luftdruck am Landeort. In verschieden Messungen wurden Spitzenwerte eines Druckanstiegs von bis zu 12 h/Pa pro Minute gemessen.

Wenn die Eustachische Röhre die nun notwendige aktive Öffnung nicht schnell genug erzeugen kann, entsteht im Mittelohr ein sehr hoher, relativer Unterdruck. Dadurch wird das Trommelfell in Richtung Mittelohr gewölbt. Dies führt zu Ohrenschmerzen, kann sich aber auch als Gesichts- oder auch Zahnschmerzen manifestieren. Im Extremfall kann es zu Blutungen oder Einrissen des Trommelfells kommen (Barotrauma).

Um hier Abhilfe zu schaffen, wurde bereits vorgeschlagen (US 5,819,745 A und EP 0 862 398 B1), eine schützende Vorkammer vor dem Trommelfell im Gehörgang durch das Einsetzen eines Gehörschutz-Körpers zu bilden. In dieser Vorkammer steigt oder fällt der Luftdruck wesentlich zeitlich verzögert gegenüber der umgebenden Atmosphäre. Der Eustachischen Röhre soll so zeitlich verlängert Gelegenheit gegeben werden, für einen Druckausgleich des Mittelohres zu sorgen, wodurch eine schmerzliche Überwölbung des Trommelfells verhindert wird. Dies wird durch ein zum Druckausgleich verwendetes, relativ kurzes, zylindrisches poröses Keramikteil versucht zu erreichen, das in der zentrischen Bohrung eines weich-elastischen Ohrenstöpsels (Gehörschutz-Körper) mit äußeren Andichtungslammellen fest eingepasst ist. Es besitzt vorzugsweise mit einem Durchmesser von 2 mm und einer Länge von 2,8 mm relativ kurze Abmessungen.

Die Herstellung eines solchen amorphen Keramikmaterials ist jedoch technologisch schwierig und finanziell aufwendig. Komgrößen und Abstand der Körner zueinander, die sog. Kavitäten, müssen in der keramischen Pulververdichtung beherrscht werden, um die geforderten engen Toleranzen der Serienproduktion konstant zu halten.

Die US 2004/0129276 A1 offenbart einen in den Gehörgang des Ohres einzusetzenden Gehörschutz wie er als Schallschutz oder an akustischen Geräten wie Hörgeräte Verwendung findet. Er besteht aus einem zylindrischen weichelastischen Schaumstoff-Stöpsel mit glatter Mantel-Außenseite und einer zentrischen Durchgangsbohrung, in der ein flexibler zylindrischer Zentralkörper fest eingebracht ist. Der Zentralkörper hat an seinem in eingestecktem Zustand äußeren Ende ein wesentlich vergrößertes Außenteil mit einem Schallverstärker, von dem aus eine schallführende zentrische Bohrung durch den Zentralkörper hindurchführt. Am Außenmantel des Zentralkörpers sind mehrere Ringlamellen (oder eine Spirallamelle) vorgesehen, die mit je einer axialen Öffnung versehen sind, wobei der Zwischenraum zwischen den äußersten Lamellen mit einer radial durch den Schallverstärker-Außenteil nach außen führenden schmalen Entlüftungsbohrung in Verbindung steht. Hierdurch ist ein relativ langer Druckausgleichskanal vorhanden. Dieser Gehörschutz ist ganz ersichtlich sehr komplex aufgebaut, mit verwinkeltem Druckausgleichskanal; zudem ist durch die glatte Außenfläche nicht ausreichend sicher abdichtend im Gehörgang fixierbar und somit nicht sinnvoll verwendbar insbesondre als z. B. Einweg-Gehörschutz für Flugreisende.

In der FR 2 588 055 A1 ist ein Filter für Hörgeräte beschrieben, der im Verlauf der schallführenden dünnen Schlauchleitung eingesetzt ist, die zwischen dem Lautsprecher oder Schallsender des Geräts und der in den Gehörganganfang einzuführenden schirmstöpselförmigen Othoplastik. Er dient dem Ausfiltern oder Bevorzugen der tiefen und/oder hohen Töne, je nach Anordnung entlang des Schlauches und besteht aus einem außen zylindrischen oder profilierten Kernstück, das axiale Schall-Durchlässe vorsieht und im Schlauch axial verschieblich festsetzbar ist, zur Anpassung der Tonqualität. Ein Druckausgleich ist weder vorgesehen, noch möglich. Somit ist dieser Filter nicht für einen druckausgleichenden Gehörschutz für Flugreisende einsetzbar.

Aufgabe der vorliegenden Erfindung ist es somit, einen Gehörschutz obiger Gattung anzugeben, der in einfacher und wirtschaftlicher Weise herstellbar ist.

Diese Aufgabe wird durch einen Gehörschutz mit den Merkmalen des Anspruchs 1 gelöst. Vorteilhafte Ausgestaltungen sind in den Unteransprüchen gekennzeichnet.

Demgemäß ist jeweils in einer zentralen Bohrung eines außen mit Abdichtlamellen versehenen Gehörschutz-Körpers, der vorzugsweise aus weichelastischem Material besteht, ein Druckregler oder eine Strömungsdrosseleinheit eingesetzt; in der Bohrung ist ein dicht ausfüllender Zentralkörper eingesetzt. Der Zentralkörper ist durch einen Zylinderstift mit einer Länge von mindestens 1,5 mal dem Durchmesser gebildet. Weiterhin ist mindestens eine Kugel in den Zentralkörper einsetzbar. Der Zylinderstift besteht aus einem luftundurchlässigem Material

In der Bohrung ist entweder eine Axialnut vorgesehen, wobei der Zylinderstift glatt ist oder die Bohrung ist glatt ausgelegt, wobei der Zylinderstift eine axiale Nut oder eine Längsabflachung besitzt.

Diese Ausgestaltung ermöglicht es, eine Mehrzahl unterschiedlicher Ausführungsformen zu bilden.

Da eine axiale Nut oder ein axialer nutförmiger Durchlass mechanisch relativ einfach herstellbar ist, mit für eine optimale Wirkung passenden Abmessungen, ist der erfindungsgemäße Gehörschutz auch insgesamt maximal wirkungsvoll verwendbar und auch wirtschaftlich herstellbar.

Von Vorteil dabei ist auch, dass die Axialnut in der zentralen Bohrung des (Ohrstöpsel-) Gehörschutz-Körpers vorgesehen ist und als Zentralkörper ein die Bohrung dicht ausfüllender Zylinderstift mit einer Länge von mindestens 1,5 mal dem Durchmesser vorgesehen ist. Durch die zylindrische Außenfläche des Zylinderstifts wird die Axialnut entsprechend zu einem schmalen axialen Durchlass verringert, dessen Querschnitt genau bestimmbar ist. Auch ist die Anfertigung eines solchen Gehörschutzes relativ einfach, da der Gehörschutz-Körper üblicherweise im Spritzgussverfahren herstellbar ist, mitsamt der Axialnut, während ein einfach herstellbarer oder bereits käuflich in entsprechenden Dimensionen erhaltbarer Zentralkörper, d. h. Zylinderstift nur einfach in die zylindrische Bohrung hineingeschoben oder hineingedrückt werden muss.

Bei einer weiteren Ausführungsform ist die Bohrung glatt ausgelegt, und ein zylindrisches Röhrchen ist in die Bohrung so eingesetzt, dass eine luftdichte Anlage der jeweiligen Mantelflächen vorhanden ist, wobei das Röhrchen mindestens die Länge des Zylinderstiftes besitzt.

Das Röhrchen ermöglicht es, eine Mehrzahl unterschiedlicher Ausführungsformen zu bilden.

So kann in das Röhrchen eine Kugel eingesetzt werden. Das Röhrchen kann auch mit mindestens zwei axial hintereinander eingeschobenen Kugeln ausgefüllt werden, ist, die ein vorbestimmtes Radialspiel zur Innenbohrung des Röhrchens aufweisen.

Statt einer Axialnut kann erfindungsgemäß an der Mantelfläche des zylindrischen Zentralkörpers eine axial verlaufende Abflachung vorgesehen sein, wodurch in sehr einfacher und kostengünstiger Weise ein entsprechender Axial-Durchlass im montierten Zustand vorhanden ist.

Nachfolgend wird die Erfindung anhand mehrer Ausführungsbeispiele unter Bezug auf die Zeichnung näher erläutert.

Es zeigen:
- Fig. 1:: eine Schnitt-Prinzipansicht eines Ohres mit eingeführtem Gehörschutz ;
- Fig. 2:: einen axialen Schnitt II-II aus Fig.3, einen Gehörschutz in erster Ausführung zeigend, mit in der zentralen, genuteten Bohrung des Gehörschutz-Körpers eingesetztem glatten Zylinderstift oder Kugel;
- Fig. 3:: einen Schnitt III-III aus Fig. 2, die Anordnung des Stiftes im Gehörschutz-Körper und dessen Fixierung veranschaulichend;
- Fig. 4:: einen Axialschnitt IV-IV ähnlich wie in Fig. 2, einen Gehörschutz in zweiter Ausführung darstellend, mit zentraler glatten Bohrung und in diese eingesetzten genuteten Zylinderstift;
- Fig. 5:: einen Radialschnitt V-V aus Fig. 4;
- Fig. 6:: einen axialen Schnitt VI-VI aus Fig. 7, als Gehörschutz in dritter fünfter Ausführung, mit in der zentralen Bohrung eingesetztem kurzen Röhrchen mit axialer Innen-Nut und darin eingesetztem Zylinderstift;
- Fig. 7:: einen Schnitt VII-VII aus Fig.6;
- Fig. 8:: einen axialen Schnitt VIII-VIII durch einen Gehörschutz in vierter Ausführung, mit in der Zentralbohrung eingesetztem glatten Röhrchen und darin eingesetztem Zylinderstift mit Axialnut;
- Fig. 9:: einen Schnitt IX-IX aus Fig. 8,
- Fig. 10:: einen axialen Schnitt X-X aus Fig. 11, einen Gehörschutz in fünfter Ausführung zeigend, mit in der Zentralbohrung eingesetztem glatten Röhrchen und darin eingesetztem Zylinderstift mit axialer Abflachung, und
- Fig. 11:: einen radialen Schnitt XI-XI aus Fig. 10.

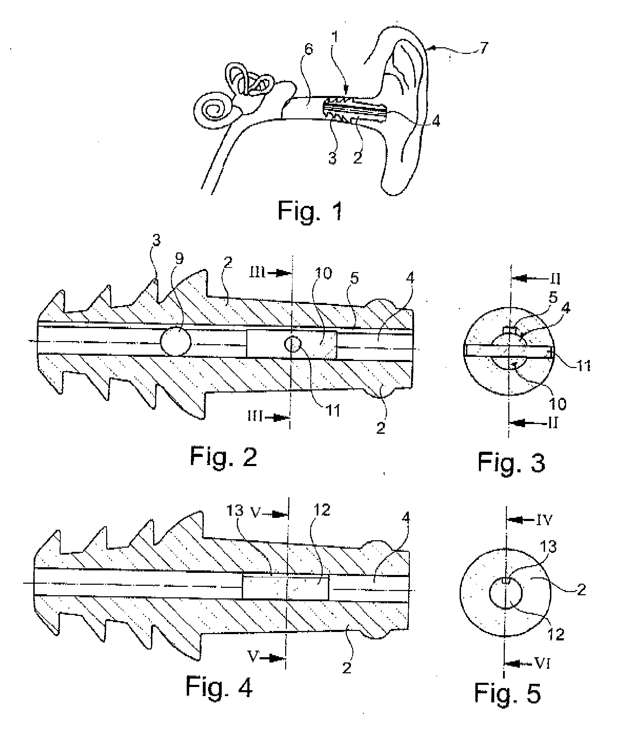

Fig. 1 zeigt einen an sich bekannten Gehörschutz 1, an dessen Körper RingLamellen 3 vorgesehen sind, der eine zentrale Innen-Bohrung 4 besitzt und der in einen Gehörgang 6 eines Ohres 7 eingesetzt ist. Es ist zu erkennen, dass der Gehörschutz im Gehörgang 6 so eingesetzt ist, dass seine Lamellen 3 einen luftdichten Abschluss bilden. Ein Luftaustausch kann hier nur noch über die zentrale Bohrung 4 erfolgen.

Bei dem in **Fig. 2** und **Fig. 3** dargestellten ersten Ausführungsbeispiel besitzt der mit Außenlamellen 3 in bekannter Weise ausgerüstete Gehörschutz-Körper 2 eine zentrale Bohrung 4, die mit einer Axialnut 5 versehen ist. In der Bohrung 4 ist ein glatter Zylinderstift 10 eingesetzt, der die Bohrung 4 über seien Umfang luftdicht verschließt. Selbstverständlich kann entweder ein Zylinderstift oder in beliebiger beliebiger Stückzahl dort angeordnet sein, wobei er/sie jeweils fest in die Bohrung eingesetzt ist/sind. Die Drosselung erfolgt durch die Axialnut 5, wobei auch mehrere Axialnuten 5 verteilt am Umfang vorgesehen sein könnten. Zudem ist hier der Zylinderstift 10 gegen Herausrutschen noch zusätzlich durch einen Radialstift 11 gesichert.

In **Fig. 4** und **Fig. 5** ist ein zweites Ausführungsbeispiel dargestellt, bei dem in der glatten zentrischen Körper-Bohrung 4 ein Zylinderstift 12 eingesetzt ist, der eine axiale Nut 13 besitzt. Die Drosselung erfolgt hier über die entsprechend dimensionierte Nut 13. Der kurze Zylinderstift hat eine Länge von z. B. mindestens 1,5 mal sein Durchmesser.

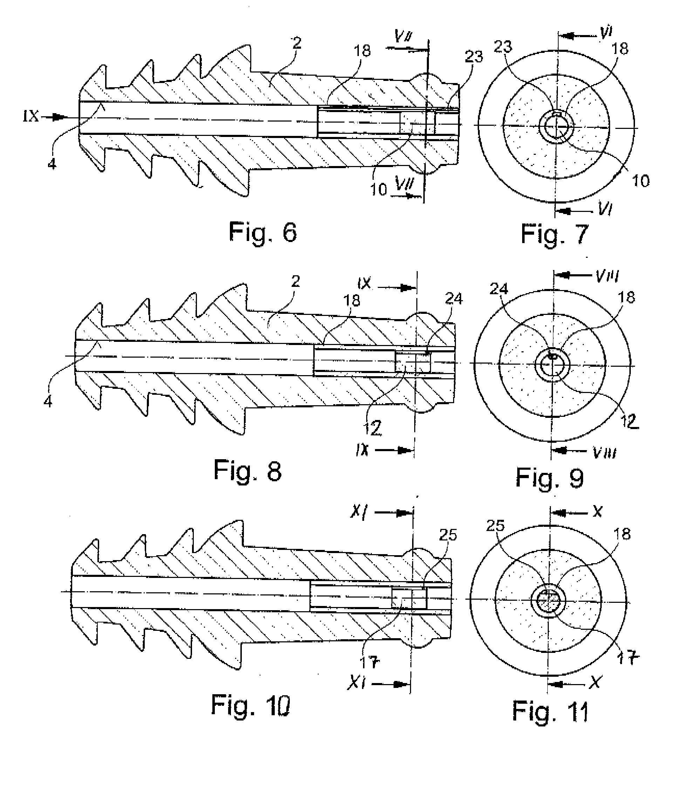

Fig. 6 und Fig. 7 zeigen einen Gehörschutz-Körper 2, in dessen glatter Bohrung 4 ein kurzes Röhrchen 18 eingesetzt ist, das eine Axialnut 23 besitzt.Im Röhrchen selbst ist ein glatter Zylinderstift 10 fest eingesetzt, so dass eine Drosselung lediglich über die Axialnut 23 erfolgen kann.

Fig. 8 und Fig. 9 zeigen ebenfalls einen Gehörschutz-Körper 2, in dessen zentraler Bohrung 4 ein kurzes Röhrchen 18 eingesetzt ist, in dem wiederum ein genuteter Zylinderstift 12 fest eingesetzt ist. Hier ist jedoch das Röhrchen 18 glattwandig ausgebildet, ohne Nuten, während der Zylinderstift 12 eine Axialnut 24 besitzt. Die Drosselung erfolgt auch hier über die Axialnut 24.

Schließlich zeigen Fig. 10 und Fig. 11 eine Ausführungsform ähnlich wie Fig. 8 und 9, nur dass hier ein Zylinderstift 17 vorhanden ist, der statt einer Axialnut eine Längs-Abflachung 25 besitzt. Die Drosselung erfolgt hier durch den axialen Durchlass, der durch die Abflachung 25 geschaffen wird.

### Bezugszeichenliste

- 1.: Gehörschutz
- 2.: Körper (Gehörschutz-)
- 3.: Lamellen
- 4.: Bohrung (Körper-/ Innen-)
- 5.: Axialnut
- 6.: Gehörgang
- 7.: Ohr
- 8.: ---
- 9.: ---
- 10.: Zylinderstift, glatt
- 11.: Stift
- 12.: Zylinderstift, genutet
- 13.: Nut
- 14.: ---
- 15.: ---
- 16.: ---
- 17.: Zylinderstift, abgeflacht
- 18.: Röhrchen (Aufnahme-, Zylinder-)
- 19.: ---
- 20.: ---
- 21.: ---
- 22.: ---
- 23.: Axialnut (Röhrchen)
- 24.: Axialnut (Stift)
- 25.: Abflachung

## Patentansprüche

1. Gehörschutz zum Druckausgleich für Flugreisende, mit
- einem eine zentrale Bohrung (4) aufweisenden Gehörschutz-Körper (2) aus weichelastischem Material, der an seiner Mantelfläche Abdichtlamellen (3) zur luftdichten Abdichtung in im Gehörgang (6) eines Ohrs (7) eingesetzten Zustand besitzt,
- einem in der Bohrung (4) vorgesehenen Druckregler oder einer Strömungsdrosseleinheit, und
- mit einem die Bohrung (4) dicht ausfüllend eingesetzten Zentralkörper,
**dadurch gekennzeichnet,**
- **dass** der Zentralkörper durch einen Zylinderstift (10, 12) mit einer Länge von mindestens 1,5 mal dem Durchmesser gebildet ist und/oder dass in den Zentralkörper mindestens eine Kugel (9) eingesetzt ist,
- **dass** der Zylinderstift aus luftundurchlässigem Material besteht,
- **dass** in der Bohrung (4) entweder eine Axialnut (5) vorgesehen ist und der Zylinderstift glatt ist oder dass die Bohrung (4) glatt ausgelegt ist und der Zylinderstift eine axiale Nut (13) oder eine Längsabflachung (25) besitzt.

2. Gehörschutz nach Anspruch 1, **dadurch gekennzeichnet,**
- **dass** die Bohrung (4) glatt ausgelegt ist, und
- ein zylindrisches Röhrchen (18) in die Bohrung (4) so eingesetzt ist, dass eine luftdichte Anlage der jeweiligen Mantelflächen vorhanden ist, und
- **dass** das Röhrchen (18) mindestens die Länge des Zylinderstiftes besitzt.

3. Gehörschutz nach Anspruch 2, **dadurch gekennzeichnet,**
- **dass** das Röhrchen (18) eine axiale Innennut (23) besitzt; und
- **dass** in das Röhrchen (18) ein zylindrischer Stift (10) mit glatter Mantelfläche abdichtend eingesetzt ist.

4. Gehörschutz nach Anspruch 3, **dadurch gekennzeichnet,**
- **dass** in das Röhrchen (18) eine Kugel (9) eingesetzt ist.

5. Gehörschutz nach Anspruch 1, **dadurch gekennzeichnet,**
- **dass** anstatt des Zylinderstiftes (10) ein Röhrchen (18) vorgesehen ist, das in die Bohrung (4) dicht eingesetzt ist, und
- **dass** das Röhrchen (18) im wesentlichen die gesamte Länge der Bohrung (4) einnimmt,
- **dass** das Röhrchen (18) mit mindestens zwei axial hintereinander eingeschobenen Kugeln (9) ausgefüllt ist, die ein vorbestimmtes Radialspiel zur Innenbohrung des Röhrchens (18) aufweisen.

6. Gehörschutz nach Anspruch 5, **dadurch gekennzeichnet,**
- **dass** im Röhrchen (18) ein axial eingedrückte Stift (19) vorgesehen ist, der die Kugeln (9) gegen axiales Herausfallen sichert.

7. Gehörschutz nach Anspruch 5 oder 6, **dadurch gekennzeichnet,**
- **dass** das Röhrchen (18) einen Einschnitt aufweist, der zwei Lappen bildet, die radial gedrückt sind und einen Axialanschlag für die Kugeln (9) bilden.

8. Gehörschutz nach Anspruch 1, **dadurch gekennzeichnet,**
- **dass** der Zylinderstift gegen Herausrutschen aus der Bohrung (4) durch einen Radialstift (11) gesichert ist.

9. Gehörschutz nach Anspruch 1, **dadurch gekennzeichnet,**
- **dass** die Bohrung (4) ein Gewindebohrung (14) besitzt, in die ein Gewindebolzen (15) eingeschraubt ist, der vorzugsweise eine axiale Abflachung (16) besitzt.

## Claims

1. Hearing protector for pressure compensation for airline passengers, comprising:
- a hearing protector body (2), which comprises a central hole (4), is made of flexible material, and has sealing lamellae (3) on the outer surface thereof for airtight sealing when used in the auditory canal (6) of an ear (7),
- a pressure regulator, which is provided in the hole (4), or a flow throttle unit, and
- a central body inserted so as to fill out the hole (4) tightly,
**characterised in that**
- the central body is formed by a cylindrical pin (10, 12) having a length of at least 1.5 times the diameter and/or **in that** at least one ball (9) is inserted into the central body,
- the cylindrical pin consists of air-impermeable material,
- either an axial groove (5) is provided in the hole (4) and the cylindrical pin is smooth or the hole (4) is formed smoothly and the cylindrical pin has an axial groove (13) or a longitudinal bevel (25).

2. Hearing protector according to claim 1, **characterised in that**
- the hole (4) is formed smoothly,
- a cylindrical tube (18) is inserted into the hole (4) in such a way that there is airtight contact between the respective outer surfaces, and
- **in that** the tube (18) has at least the length of the cylindrical pin.

3. Hearing protector according to claim 2, **characterised in that** the tube (18) has an axial internal groove (23); and
- **in that** a cylindrical pin (10) having a smooth outer surface is tightly inserted into the tube (18).

4. Hearing protector according to claim 3, **characterised in that**
- a ball (9) is inserted into the tube (18).

5. Hearing protector according to claim 1, **characterised in that**
- a tube (18) is provided instead of the cylindrical pin (10) and is inserted tightly into the hole (4),
- the tube (18) takes up substantially the entirely length of the hole (4), and
- the tube (18) is filled up with at least two balls (9), inserted in axial succession, which have a predetermined radial play with respect to the internal hole of the tube (18).

6. Hearing protector according to claim 5, **characterised in that**
- an axially impressed pin (19) is provided in the tube (18) and secures the balls (9) against falling out axially.

7. Hearing protector according to either claim 5 or claim 6, **characterised in that**
- the tube (18) comprises an incision which forms two lobes which are pressed radially and which form an axial stop for the balls (9).

8. Hearing protector according to claim 1, **characterised in that**
- the cylindrical pin is secured against sliding out of the hole (4) by a radial pin (11).

9. Hearing protector according to claim 1, **characterised in that**
- the hole (4) has a threaded hole (14), into which a threaded bolt (15) is screwed which preferably has an axial bevel (16).

## Revendications

1. Protection auditive destinée à compenser la différence de pression pour passagers d'avion, comportant :
- un corps de protection auditive (2) présentant un alesage central (4) et constitué d'un matériau flexible qui comporte, sur son enveloppe, des lamelles d'étanchéité (3) pour obtenir une étanchéité à l'air dans l'état de mise en place dans le conduit auditif (6) d'une oreille (7),
- un régulateur de pression prévu dans l'alesage (4) ou une unité limitant le débit d'air, et
- comportant un corps central mis en place pour remplir l'alesage (4) de manière étanche,
**caractérisée**
- **en ce que** le corps central est formé d'une cheville cylindrique (10, 12) d'une longueur représentant au moins 1,5 fois le diamètre et/ou en ce que dans le corps central au moins une bille (9) est mise en place,
- **en ce que** la cheville cylindrique est constituée d'un matériau étanche à l'air,
- **en ce que**, dans l'alesage (4), soit une rainure axiale (5) est prévue et la cheville cylindrique est lisse, soit l'alesage (4) est configuré lisse et la cheville cylindrique possède une rainure axiale (13) ou un méplat longitudinal (25).

2. Protection auditive selon la revendication 1, **caractérisée**
- **en ce que** l'alesage (4) est configuré lisse, et
- un petit tuyau cylindrique (18) est mis en place dans l'alesage (4) de telle sorte qu'un appui étanche à l'air des enveloppes respectives soit présent, et
- **en ce que** le petit tuyau (18) possède au moins la longueur de la cheville cylindrique.

3. Protection auditive selon la revendication 2, **caractérisée**
- **en ce que** le petit tuyau (18) possède une rainure intérieure axiale (23) ; et
- **en ce que**, dans le petit tuyau (18), une cheville cylindrique (10) avec une enveloppe lisse est mise en place de manière étanche.

4. Protection auditive selon la revendication 3, **caractérisée**
- **en ce que**, dans le petit tuyau (18), une bille (9) est mise en place.

5. Protection auditive selon la revendication 1, **caractérisée**
- **en ce que**, au lieu de la cheville cylindrique (10), un petit tuyau (18) est prévu et mis en place de manière étanche dans l'alesage (4), et
- **en ce que** le petit tuyau (18) occupe essentiellement toute la longueur de l'alesage (4),
- **en ce que** le petit tuyau (18) est rempli d'au moins deux billes insérées axialement l'une derrière l'autre (9) et qui présentent un jeu radial prédéterminé par rapport à l'alesage intérieur du petit tuyau (18).

6. Protection auditive selon la revendication 5, **caractérisée**
- **en ce que** dans le petit tuyau (18) une cheville (19) enfoncée axialement, qui empêche les billes (9) de ressortir axialement, est prévue.

7. Protection auditive selon la revendication 5 ou 6, **caractérisée**
- **en ce que** le petit tuyau (18) présente une encoche qui forme deux languettes, lesquelles sont pressées radialement et forment une butée axiale pour les billes (9).

8. Protection auditive selon la revendication 1, **caractérisée**
- **en ce que** la cheville cylindrique est empêchée de ressortir de l'alesage (4) par une cheville radiale (11).

9. Protection auditive selon la revendication 1, **caractérisée**
- **en ce que** l'alesage (4) possède un trou fileté (14) dans lequel un boulon fileté (15) est vissé et comporte de préférence un méplat axial (16).
